# EUROPÄISCHE PATENTSCHRIFT

(11) **EP 1 392 226 B1**
(45) Veröffentlichungstag und Bekanntmachung des Hinweises auf die Patenterteilung: **18.02.2009**
(21) Anmeldenummer: 02776501.5
(22) Anmeldetag: 05.05.2002
(51) Int. Cl.: A61K 8/04, A61K 8/73, A61K 8/81, A61K 8/86, A61Q 5/00, A61Q 5/06

(54) **ZWEIPHASIGES HAARGEL**
TWO-PHASE HAIR GEL
GEL CAPILLAIRE A DEUX PHASES

(30) Priorität: 02.06.2001 DE 10127104
(43) Veröffentlichungstag der Anmeldung: 03.03.2004
(73) Patentinhaber: Wella Aktiengesellschaft, 64274 Darmstadt (DE)
(72) Erfinder: KALBFLEISCH, Axel, 64295 Darmstadt (DE); KRAUSE, Thomas, 64297 Darmstadt (DE); SENDELBACH, Gerhard, 64297 Darmstadt (DE); BEYER, Angelika, 63857 Waldaschaff (DE); BAECKER, Sabine, 65428 Rüsselsheim (DE); PFAFFERNOSCHKE, Matthias, 6208 Oberkirch (CH)
(86) Internationale Anmeldenummer: PCT/EP2002/004807
(87) Internationale Veröffentlichungsnummer: WO 2002/098371

(56) Entgegenhaltungen:
- EP-A- 0 923 931
- EP-A- 1 161 935
- WO-A-98/08601
- WO-A-99/51716
- WO-A1-94/12151
- WO-A2-98/50000
- DE-A- 2 220 662
- DATABASE CA CHEMICAL ABSTRACTS SERVICE, COLUMBUS, OHIO, US; gefunden im STN Database accession no. 142: 120 098 & KR 2001 018 775 A (LG CHEM INVESTMENT) 15. März 2001 (2001-03-15)

## Beschreibung

Gegenstand der Erfindung ist ein Haarbehandlungsmittel in Form eines zweiphasigen Gels, enthaltend zerkleinerte Partikel eines festen und formstabilen Gels in einer fluiden Matrix.

Um dem menschlichen Haar Festigung, Halt, Modellierbarkeit oder Glanz zu geben oder um eine erstellte Frisur zu stabilisieren, werden Haarbehandlungsmittel in Form von verdickten Präparaten wie z.B. Gelen, Haarcremes oder Haarwachsen eingesetzt. Derartige Produkte haben eine hochviskose, aber dennoch fliessfähige bis halbfeste oder cremeförmige einheitliches homogene Konsistenz. Neben den oben genannten primären Produktleistungen sind auch Haptik und Konsistenz von wesentlicher Bedeutung für die Produktqualität haarkosmetischer Erzeugnisse. Von Interesse sind insbesondere klare Produkte sowie solche mit neuartigen Produktkonsistenzen und mit neuen, ausgefallenen Eigenschaften. Eine solche Eigenschaft ist z.B. ein bei Anwendung des kosmetischen Mittels auftretender besonderer sensorischer, insbesondere taktiler Effekt, d.h. eine besondere Haptik. Durch diese neuen, ausgefallenen Eigenschaften sollen die primären Produktleistungen allerdings möglichst nicht beeinträchtigt, sondern idealerweise sogar noch verstärkt werden. Es muss ausserdem eine leichte, unproblematische Anwendbarkeit, insbesondere eine gute Verteilbarkeit im Haar gewährleistet sein.

Die der Erfindung zugrunde liegende Aufgabe bestand darin, kosmetische Mittel mit neuen, ausgefallenen haptischen Eigenschaften zur Verfügung zu stellen. Die Mittel sollten nach Möglichkeit klar sein und die typischen Eigenschaften eines Haarwachses oder einer Haarpomade wie z.B. Modellierbarkeit, Halt, Glanz etc. aufweisen. Die Mittel sollen ausserdem gute Anwendungseigenschaften besitzen, insbesondere gut verreibbar und gut auf dem Haar verteilbar sein. Insbesondere bestand die Aufgabe darin, ein gut im Haar verteilbares Haargel mit festen Partikeln zur Verfügung zu stellen.

Aus der EP 0 923 931 sind feste kosmetische Mittel bekannt, welche mindestens 2% kappa-Carrageenan in Kombination mit bestimmten Hydrokolloiden auf natürlicher Basis enthalten und im wesentlichen als Körperpflegestifte (Sticks, Lippenstifte) eingesetzt werden. Bei diesen Mitteln sind relativ hohe Mengen an Carrageenan erforderlich, um die notwendige Festigkeit zu erreichen. Derartige Mittel sind zur Haarbehandlung nicht geeignet, da sich aufgrund des hohen Carrageenangehaltes (größer 2 Gew.%) unschöne, sichtbare Rückstände auf dem Haar bilden können. Bei Herabsetzung der Carrageenanmenge ist eine ausreichende Festigkeit des Produktes nicht mehr gegeben.

Es wurde nun gefunden, dass die Aufgabe gelöst wird durch ein Haarbehandlungsmittel in Form eines zweiphasigen Gels, wobei die eine Phase aus Partikeln eines festen und formstabilen Gels besteht und Carragenaan als Gelbildner enthält und die andere Phase eine fluide Matrix für die festen Partikel ist und wobei die fluide Phase mindestens einen mehrwertigen Alkohol oder mindestens einen flüssigen Polyether eines mehrwertigen Alkohols enthält, dadurch gekennzeichnet, dass der mittlere Durchmesser der festen Gelpartikel mindestens 1 mm beträgt und/oder dass das Haarbehandlungsmittel zusätzlich mindestens einen Stoff enthält, der ausgewählt ist aus haarfestigenden nichtionischen, anionischen, zwitterionischen und amphoteren Polymeren, amphiphilen Assoziatiwerdickern, Cellulosederivaten, Zuckern und Treibmitteln, wobei die Zucker Mono- oder Disaccharide sind.

Zahnpasten beschrieben, welche niedermolekulares Polyethylenglykol enthalten und mit Teilchen aus Siliciumdioxidgel einer Teilchengröße von 1 bis 20 µm verdickt sein können.

Es wurde nun gefunden, dass die Aufgabe gelöst wird durch ein Haarbehandlungsmittel in Form eines zweiphasigen Gels, wobei die eine Phase aus Partikeln eines festen und formstabilen Gels besteht und die andere Phase eine fluide Matrix für die festen Partikel ist und wobei die fluide Phase mindestens einen mehrwertigen Alkohol oder mindestens einen flüssigen Polyether eines mehrwertigen Alkohols enthält, dadurch gekennzeichnet, dass der mittlere Durchmesser der festen Gelpartikel mindestens 1 mm beträgt und/oder dass das Haarbehandlungsmittel zusätzlich mindestens einen Stoff enthält, der ausgewählt ist aus haarfestigenden nichtionischen, anionischen, zwitterionischen und amphoteren Polymeren, amphiphilen Assoziatiwerdickern, Cellulosederivaten, Zuckern und Treibmitteln. Die Menge an fester Gelphase beträgt vorzugsweise von 30 bis 95 Gew.%, besonders bevorzugt von 50 bis 85 Gew.% des gesamten Mittels und die Menge an fluider Matrix dementsprechend vorzugsweise 5 bis 70 Gew.%, besonders bevorzugt 15 bis 50 Gew.%.

Die in der fluiden Phase enthaltenen Flüssigkeiten sind vorzugsweise ausgewählt aus Alkylenglykolen, Glycerin und flüssigen Polyalkylenglykolen, wobei die Alkylengruppen vorzugsweise 2 bis 5 C-Atome aufweisen. Geeignet sind beispielsweise Ethylenglykol, Propylenglykole, insbesondere 1,2-Propylenglykol, Butylenglykol, Pentandiole und bei 25°C flüssige Polyethylenglykole, Polypropylenglykole, Polyethylenglykol-C1-4-alkylether, Polypropylenglykol-C1-4-alkylether, Ethylenglykol/Propylenglykol Copolymere oder Polyoxyethylen-polyoxypropylen-C1-4-alkylether. Geeignete Polyethylenglykole haben ein Molekulargewicht zwischen 150 und 700, vorzugsweise zwischen 170 und 300 g/mol. Geeignete niedrigmolekulare Polyethylenglykole sind solche der Formel H(OCH₂CH₂)ₙOH mit n = 4 bis 14, vorzugsweise mit n = 4 bis 8. Geeignete Polyethylenglykole haben die INCI-Bezeichnungen PEG-4 (n = 4), PEG-6(n = 6), PEG-7(n = 7), PEG-8 (n = 8), PEG-9 (n = 9), PEG-10 (n = 10), PEG-12 (n = 12) und PEG-14 (n = 14). Ein geeignetes Handelsprodukt ist z.B. Polyglykol 200 mit einem Molekulargewicht von 190 bis 210 der Firma Clariant. Besonders bevorzugt sind Glycerin, PEG-4 und PEG-6.

Die feste Phase des erfindungsgemäßen Produktes wird aus einem festen und formstabilen Gel gebildet. Fest und formstabil im Sinne der Erfindung sind insbesondere solche Zusammensetzungen, für die der Widerstand gegen Kompression unter Normbedingungen (20°C, 65% rel. Luftfeuchte) mindestens 0,15 N, vorzugsweise 0,30 bis 2,0 N, besonders bevorzugt 0,40 bis 1,5 N beträgt, gemessen durch Penetration des festen Gels mit einem zylindrischen Stempel eines Durchmessers von 8 mm und wobei die Penetration mit einer Geschwindigkeit von 0,5 mm/sec bis zu einer Kompressionstiefe von 1 mm erfolgt und anschließend der Stempel mit einer Geschwindigkeit von 0,5 mm/s wieder zurückgeführt wird. Die Formstabilität des Gels besteht mindestens bei Raumtemperatur (20°C) und darunter, vorzugsweise bei Temperaturen bis 30°C, besonders bevorzugt bei Temperaturen bis 35°C.

Als Gelbildner für die feste Gelphase wird Carrageenan eingesetzt. Normalerweise ist eine Carrageenanmenge von mindestens etwa 3 Gew.% in Wasser erforderlich, um zu einem festen und formstabilen Gel zu gelangen. Zur Vermeidung von sichtbaren Rückstandsbildungen auf dem Haar ist es aber empfehlenswert, die Carrageenanmenge zu reduzieren. Die erforderliche Festigkeit des Gels kann dabei erreicht werden, indem mindestens ein haarfestigendes Polymer und/oder mindestens 15 Gew.% mindestens eines ein- oder mehrwertigen Alkohols und/oder mindestens ein Calcium- oder Kaliumionen enthaltendes Salz zur Herstellung der festen Gelphase zugesetzt werden. Derartige Zusammensetzungen zeigen eine ausreichende Festigkeit der festen Gelphase bereits mit geringen Carrageenanmengen (z.B. unterhalb 2 Gew.%). Hierdurch ist es möglich, die Zusammensetzungen zur Haarbehandlung, insbesondere bei Zusatz von haarfestigenden Polymeren als Haarstylingmittel zur Haarfestigung einzusetzen, da das Problem der unerwünschten Rückstandsbildung gelöst ist.

In einer bevorzugten Ausführungsform liegt die feste Gelphase in Form eines festen und formstabilen Gels vor mit einem Gehalt an einer Kombination von
(A) Carrageenan und
(B) mindestens einem haarfestigenden Polymer
in einer wasserhaltigen Grundlage. Carrageenan und das haarfestigende Polymer sind in einer solchen Menge enthalten, dass das Gel bei Raumtemperatur (20°C) fest und formstabil ist.

### Carrageenan

Geeignete Gelbildner sind Carrageenan, insbesondere kappa- oder iota-Carrageenan. Besonders bevorzugt ist kappa-Carrageenan oder ein kappa-Carrageenan enthaltendes Carrageenan-Gemisch. Besonders gut geeignet ist beispielsweise ein Carrageenan mit mittlerem Molekulargewicht wie Sea Kem^{®} CM 611 der Firma FMC Corporation. Der Gelbildner wird in einer solchen Menge eingesetzt, dass die feste Phase des Mittels bei Raumtemperatur (20°C) in Form eines festen, formstabilen Gels vorliegt. Geeignete Mengen sind z.B. solche von 0,5 bis 5 Gew.%, vorzugsweise von 1 bis kleiner 2,5 Gew.%, besonders bevorzugt von 1,3 bis kleiner oder gleich 2 Gew.%. Die Festigkeit kann sich dabei teilweise erst nach einiger Zeit, d.h. im Verlaufe von zwei bis drei Tagen einstellen. Die Verfestigung des Gels kann aber dadurch beschleunigt werden, dass der Gelbildner zunächst in Wasser gegebenenfalls unter Erwärmen auf etwa 80°C gelöst und anschließend schnell mittels zusätzlicher, externer Kühlung auf mindestens 50 bis 55°C oder darunter abgekühlt wird.

### Calcium- und Kaliumionen

Die Calcium- und/oder Kaliumionen werden in Form von wasserlöslichen Salzen, z.B. den Halogeniden, Sulfaten etc., von denen die Chloride bevorzugt sind, eingesetzt, wobei die Menge der Salze vorzugsweise 0,2 bis 1 Gew.%, besonders bevorzugt 0,4 bis 0,8 Gew.% beträgt. Kaliumionen sind bevorzugt, da sich hiermit klarere, ungetrübte Gele herstellen lassen.

### Haarfestigende Polymere

Es hat sich gezeigt, dass feste Gele aus reinem Carrageenan auch ohne zusätzliche haarfestigende Polymere eine gewisse haarfestigende Wirkung besitzen. Es sind für eine ausreichende Haarfestigung aber relativ hohe Carrageenanmengen erforderlich, was zu der unerwünschten Nebenwirkung von sichtbaren Rückständen auf dem Haar führt. Bei einer Absenkung der Carrageenanmenge gehen sowohl die ausreichende Haarfestigung als auch die Festigkeit und die Formstabilität des Gels verloren. Das erfindungsgemäße Mittel enthält daher mindestens ein zusätzliches, synthetisches oder natürliches haarfestigendes Poymer. Dieses kann in der fluiden Matrix oder vorzugsweise in der festen Gelphase enthalten sein.

Das zusätzliche haarfestigende Polymer liegt vorzugsweise in einer Menge von 0,1 bis 30 Gew.%, besonders bevorzugt.von 0,5 bis 15 Gew.% vor. Das haarfestigende Polymer ist nichtionisch, anionisch, zwitterionisch oder amphoter. Besonders bevorzugt sind Polymere, welche keine kationischen Gruppen enthalten, d.h. anionische, nichtionische und amphotere Polymere. Unter synthetischen Polymeren werden solche Polymere verstanden, welche rein synthetischen, nicht natürlichen Ursprungs sind, insbesondere solche, die durch radikalische Polymerisation aus ethylenisch ungesättigten Monomeren oder durch Polykondensation herstellbar sind. Bevorzugt sind insbesondere solche Polymere, die eine ausreichende Löslichkeit oder Dispergierbarkeit in Wasser, Alkohol oder Wasser/Alkohol-Gemischen besitzen, um in dem erfindungsgemäßen Mittel auf wässriger Basis in gelöster oder homogen dispergierter Form vorzuliegen. Unter haarfestigenden Polymeren werden erfindungsgemäß solche Polymere verstanden, die bei Anwendung in 0,01 bis 5%iger wässriger, alkoholischer oder wässrigalkoholischer Lösung in der Lage sind, auf dem Haar einen Polymerfilm abzuscheiden und das Haar zu festigen.

Besonders bevorzugt werden in dem erfindungsgemäßen Gel filmbildende, haarfestigende, nichtionische, anionische oder amphotere Polymere eingesetzt. Geeignete nicht-ionische Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind. Geeignet sind z.B. Homopolymere des Vinylcaprolactams, des Vinylpyrrolidons oder des N-Vinylformamids. Weitere geeignete synthetische filmbildende, nicht-ionische, haarfestigende Polymere sind z.B. Copolymerisate aus Vinylpyrrolidon und Vinylacetat, Terpolymere aus Vinylpyrrolidon, Vinylacetat und vinylpropionat, Polyacrylamide, die z.B. unter den Handelsbezeichnungen Akypomine^{®} P 191 von der Firma CHEM-Y, Emmerich, oder Sepigel^{®} 305 von der Firma Seppic vertrieben werden; Polyvinylalkohole, die z.B. unter den Handelsbezeichnungen Elvanol^{®} von Du Pont oder Vinol^{®} 523/540 von der Firma Air Products vertrieben werden sowie Polyethylenglykol/Polypropylenglykol Copolymere, die z.B. unter den Handelsbezeichnungen Ucon^{®} der Union Carbide vertrieben werden. Besonders bevorzugt sind Polyvinylpyrrolidon, Polyvinylcaprolactam und deren Copolymere mit mindestens einem weiteren nichtionischen Monomer, insbesondere Polyvinylpyrrolidon/ Vinylacetat Copolymere.

Geeignete anionische haarfestigende Polymere sind synthetische Homo- oder Copolymere aus Säuregruppen enthaltenden Monomereinheiten, welche gegebenenfalls mit Comonomeren, die keine Säuregruppen enthalten, copolymerisiert sind. Die Säuregruppen sind vorzugsweise ausgewählt aus -COOH, -SO₃H, -OSO₃H, -OPO₂H und-OPO₃H₂, von denen die Carbonsäuregruppen bevorzugt sind. Die Säuregruppen können unneutralisiert, teilweise oder vollständig neutralisiert vorliegen. Sie liegen vorzugsweise zu 50 bis 100% in anionischer bzw. neutralisierter Form vor. Als Neutralisationsmittel können für kosmetische Zwecke geeignete organische oder anorganische Basen verwendet werden. Beispiele für Basen sind Aminoalkohole wie z.B. Aminomethylpropanol (AMP), Triethanolamin, Monoethanolamin oder Tetrahydroxypropylethylendiamin und Ammoniak, NaOH und andere. Geeignete Monomere sind ungesättigte, radikalisch polymerisierbare Verbindungen, welche mindestens eine Säuregruppe tragen, insbesondere Carboxyvinylmonomere. Geeignete Säuregruppen enthaltende Monomere sind z.B. Acrylsäure, Methacrylsäure, Crotonsäure, Maleinsäure bzw. Maleinsäureanhydrid oder deren Monoester.

Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie zum Beispiel Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1 bis C7-Alkylgruppen, besonders bevorzugt C1- bis C3-Alkylgruppen sind.

Geeignete Polymere mit Säuregruppen sind insbesondere Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes natürliches Polymer ist beispielsweise Schellack.

Bevorzugte Polymere mit Säuregruppen sind vernetzte oder unvernetzte Vinylacetat/Crotonsäure Copolymere. Ebenso bevorzugt sind partialveresterte Copolymere zwischen Vinylmethylether und Maleinsäureanhydrid. Weitere bevorzugte anionische Polymere sind z.B. Terpolymere aus Acrylsäure, Alkylacrylat und N-Alkylacrylamid, insbesondere Acrylsäure/Ethylacrylat/N-t-Butylacrylamid Terpolymere oder Terpolymere aus Vinylacetat, Crotonat und Vinylalkanoat, insbesondere Vinylacetat/Crotonat/Vinylneodecanoat Copolymere sowie Copolymere aus Acryl- oder Methacrylsäure und Acryl-oder Methacrylsäurealkylestern, wobei die Alkylgruppen vorzugsweise 1 bis 7 C-Atome enthalten.

Geeignete haarfestigende amphotere Polymere sind Polymere, welche neben sauren oder anionischen Gruppen als weitere funktionelle Gruppen basische oder kationische Gruppen, insbesondere primäre, sekundäre, tertiäre oder quaternäre Amingruppen enthalten. Beispiele hierfür sind Copolymere gebildet aus Alkylacrylamid (insbesondere Octylacrylamid), Alkylaminoalkylmethacrylat (insbesondere t-Butylaminoethylmethacrylat) und zwei oder mehr Monomeren bestehend aus Acrylsäure, Methacrylsäure oder deren Ester, wie sie zum Beispiel unter dem Handelsnamen Amphomer^{®} oder Amphomer^{®} LV-71 der Firma NATIONAL STARCH, USA erhältlich sind. Weitere Beispiele für als Komponente (B) geeignete Copolymere mit Säuregruppen sind Copolymere von Acrylsäure, Methylacrylat und Methacrylamidopropyltrimethylammoniumchlorid (INCI-Bezeichnung: Polyquaternium-47), wie sie beispielswiese von der Firma Calgon unter der Handelsbezeichnung Merquat^{®} 2001 vertrieben werden, Copolymere aus Acrylamidopropyltrimethylammoniumchlorid und Acrylaten, wie sie beispielsweise von der Firma Stockhausen unter der Handelsbezeichnung W 37194 erhältlich sind oder Copolymere aus Acrylamid, Acrylamidopropyltrimethylammoniumchlorid, 2-Amidopropylacrylamidsulfonat und Dimethylaminopropylamin (INCI-Bezeichnung: Polyquaternium-43), wie sie beispielsweise von der Firma Societe Francaise Hoechst unter der Handelsbezeichnung

Bozequat^{®} 4000 vertrieben werden. Geeignet sind auch Polymere mit Betaingruppen tragenden Monomeren wie z.B. Copolymere aus Methacryloylethylbetain und zwei oder mehr Monomeren von Acrylsäure oder deren einfachen Estern, bekannt unter der INCI-Bezeichnung Methacryloyl Ethyl Betaine/Acrylates Copolymer.

### Wässrige Grundlage

Das erfindungsgemäße feste Gel wird bevorzugt in einer wässrigen Grundlage konfektioniert. Hierunter wird entweder ein rein wässriges oder ein wässrig-alkoholisches Medium mit vorzugsweise bis zu 40 Gew.% Alkoholen verstanden. Als Alkohole können insbesondere die für kosmetische Zwecke üblicherweise verwendeten niederen ein- oder mehrwertigen Alkohole mit 1 bis 5 Kohlenstoffatomen wie z.B. Ethanol, Isopropanol, Ethylenglykol, Glycerin und Propylenglykole, insbesondere 1,2-Propylenglykol, enthalten sein. Typische Wassergehalte sind 55 bis 95, bevorzugt 65 bis 80 Gew.%, typische Alkoholgehalte sind 0 bis 30, vorzugsweise 1 bis 25 Gew.%. Bei Alkoholgehalten über 40 Gew.% besteht die Gefahr, dass das Carrageenan ausfällt.

Besonders vorteilhaft ist die Verwendung von mindestens 15 Gew.% Alkohol, da hierbei eine ausreichende Festigkeit und Formbeständigkeit des Gels bereits mit Carrageenanmengen von weniger als den sonst mindestens erforderlichen 2,5 bis 3 Gew.%, insbesondere mit weniger als 2 Gew.% erreicht werden können. Außerdem ist bei Verwendung von mindestens 15 Gew.% Alkohol ein zusätzliches Konservierungsmittel nicht unbedingt erforderlich.

Ein besonderer Vorteil des erfindungsgemäßen Gels ist die Viskositätsstabilität über einen weiten pH-Bereich von 1 bis 14. Besonders bevorzugt ist der pH-Bereich zwischen 2,5 und 8.

### Konservierungsmittel

Da das für das erfindungsgemäße Gel einzusetzende Carrageenan ein Polymer natürlichen Ursprungs auf Saccharidbasis ist, sind besondere Anforderungen an die Konservierung des erfindungsgemäßen Mittels zu stellen, um eine längere Haltbarkeit zu gewährleisten. Als besonders geeignete Konservierungsmittel haben sich Parabene, beispielsweise Methylparaben erwiesen. Bei Ethanolgehalten um etwa 15 Gew.% und darüber ist ein zusätzliches Konservierungsmittel nicht unbedingt erforderlich.

### Zucker

Zur Verbesserung der Klarheit und Transparenz des erfindungsgemäßen Mittels ist es zweckmässig, dass zusätzlich mindestens ein Zucker enthalten ist. Dieser befindet sich vorzugsweise in der festen Gelphase. Geeignete Zucker sind insbesondere Mono- und Disaccharide wie z.B. Glukose, Galaktose, Fructose, Maltose, Lactose oder Saccharose. Typische Einsatzmengen sind 0,01 bis 5, vorzugsweise 0,05 bis 1 Gew.%. Bevorzugt werden fertige Mischungen von Carrageenan und Zucker wie z.B. der Rohstoff Seakem CM 611, bei dem es sich um eine Mischung von Carrageenan und Dextrose handelt.

### Zusätze für bessere Verreib- und Verteilbarkeit

Zur Verbesserung der Verreibarkeit in den Händen bzw. zur Verbesserung der Verteilbarkeit auf dem Haar oder zur weiteren Optimierung der Konsistenz enthält das erfindungsgemäße Mittel vorzugsweise weitere Verdicker oder Gelbildner. Diese können in der flüssigen Matrix oder vorzugsweise in der fesen Gelphase enthalten sein. Geeignet sind z.B. Carboxyvinylpolymere, insbesondere Polyacrylate wie z.B. die verschiedenen Carbopol-Typen, außerdem Polyglykole, Cellulosederivate, insbesondere Hydroxyalkylcellulosen sowie anorganische Verdicker wie z.B. natürliche oder synthetische Bentonite. Typische Einsatzkonzentrationen der zusätzlichen Gelbildner und Verdicker sind von etwa 0,2 bis 10,0 Gew.%, vorzugsweise 1 bis 5 Gew.%.

Geeignete Substanzen, welche die Verreibbarkeit des Gels oder die Verteilbarkeit des Gels auf Haaren erleichtern, sind Xanthan Gum und Cellulosederivaten, wie sie in der EP 0 923 931 beschrieben werden. Hierbei handelt es sich um in heissem Wasser lösliche Hydrokolloide, insbesondere um Carboxymethylcellulose und Hydroxyethylcellulose.

Überraschenderweise wurde gefunden, dass eine besonders gute Verreibarkeit und Verteilbarkeit von festen Gelen auf Carrageenan-Basis erzielt wird bei Verwendung von amphiphilen Assoziativverdickern. Geeignete amphiphile Assoziativverdicker sind nichtionische Polymere, welche sowohl hydrophile als auch hydrophobe Gruppen enthalten. Assoziativverdicker sind wasserlösliche Polymere und haben tensidartige hydrophobe Bestandteile, welche in der Lage sind, sich in einem hydrophilen, insbesondere wässrigen Medium sowohl mit sich selbst als auch mit anderen hydrophoben Stoffen zu assoziieren, d.h. in Wechselwirkung zu treten. Durch das daraus resultierende assoziative Netzwerk wird das Medium verdickt oder geliert. Typischerweise werden Assoziativverdicker hergestellt durch Polymerisation von Polyethylenoxid-Prepolymeren und mindestens zweifach funktionellen, polykondensierbaren Stoffen wie z.B. Isocyanaten, wobei Mono- oder Diole mit großen Aryl-, Alkyl- oder Aryl/Alkyl-Gruppen eingebaut werden, um die hydrophobe Modifikation bereitzustellen. Bevorzugte Assoziativverdicker sind daher hydrophob modifizierte Polyalkylenglykole. Hierbei wird der hydrophile Bestandteil durch Polyoxyalkyleneinheiten, vorzugwseise Polyoxyethylenaber auch Polyoxypropyleneinheiten oder deren Gemisch gebildet. Der hydrophobe Bestandteil wird vorzugsweise aus Kohlenwasserstoffgruppen, z.B langkettigen Alkylgruppen, Alkylaryl- oder Arylalkylgruppen gebildet.

Besonders bevorzugte Assoziativverdicker sind hydrophob modifizierte Aminoplast-Polyether Copolymere. Bezüglich deren Struktur und Herstellung wird auf die WO 96/40815 verwiesen. In der WO 96/40815 werden wasserdispergierbare oder wasserlösliche Copolymere beschrieben, welche die Reaktionsprodukte sind einer säurekatalysierten Polykondensation von mindestens zweifach funktionellen Aminoplastmonomeren und mindestens zweifach funktionellen Alkylenpolyethern sowie einfach funktionellen Verbindungen mit hydrophoben Gruppen. Geeignete Aminoplaste sind der Figur 1 der WO 96/40815 zu entnehmen. Besonders bevorzugt sind die Glycolurilderivate der Formel X der WO 96/40815. Geeignete Alkylenpolyether sind der Figur 2 der WO 96/40815 zu entnehmen. Bevorzugte Alkylenpolyether sind Polyethylenoxiddiole. Diese können einen Ethoxylierungsgrad von 20 bis 500, vorzugsweise 50 bis 350, besonders bevorzugt von 100 bis 250 haben. Geeignete einfach funktionelle Verbindungen mit hydrophoben Gruppen sind diejenigen der Formel XIV der WO 96/40815.

Erfindungsgemäß geeignete Assoziativverdicker sind ausgewählt aus Polymeren der allgemeinen Formel (I) 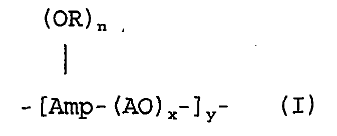 wobei Amp ein Aminoplastmonomer oder den Rest eines Aminoplastoligomers oder eines Aminoplastpolymers bedeutet, AO für eine Alkylenoxidgruppe steht, R für Wasserstoff, C1-C4-Alkyl oder C1-C4-Acyl steht, x und y Zahlen größer 1 sind und n eine positive Zahl ist, die der Anzahl der freien Valenzen von Amp entspricht. Besonders bevorzugt sind die Reaktionsprodukte der säurekatalysierten Polykondensation von (a) Glykolurilen der allgemeinen Formel (II), 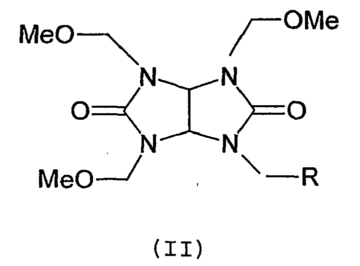 wobei R für H oder vorzugsweise für OMe steht mit (b) Polyethylenoxiddiolen eines Ethoxylierungsgrades von 20 bis 500, vorzugsweise 50 bis 350, besonders bevorzugt von 100 bis 250 sowie (c) eines gegebenenfalls ethoxylierten hydrophoben Alkohols, Alkylphenols, Thiols, Carboxamids, Carbamats oder einer hydrophoben Carbonsäure, wie sie auf den Seiten 17 bis 19 der WO 96/40815 beschrieben sind. Besonders bevorzugtes Glykoluril ist 1,3,4,6-Tetramethoxymethylglycoluril.

Geeignete Assoziativverdicker sind solche mit den INCI-Bezeichnungen Polyether-1, PEG-180/Octoxynol-40/TMMG Copolymer und PEG-180/Laureth-50/TMMG Copolymer und werden von der Firma Süd-Chemie vertrieben unter den Handelsbezeichnungen Pure-Thix^{®} HH, L, M oder TX.

### Optionale Zusatzbestandteile

Das erfindungsgemäße Mittel kann darüber hinaus in der festen Gelphase und/oder in der fluiden Matrix die für Haarbehandlungsmittel üblichen Zusatzbestandteile enthalten, z.B Netzmittel oder Emulgatoren in einer Menge von 0,1 bis 15 Gew.%; Feuchthaltemittel; Parfümöle in einer Menge von 0,1 bis 0,5 Gew.%; bakterizide und fungizide Wirkstoffe wie zum Beispiel 2,4,4-Trichlor-2-hydroxydiphenylether oder Methylchlorisothiazolion, in einer Menge von 0,01 bis 1,0 Gew.%; Puffersubstanzen, wie z.B. Natriumcitrat oder Natriumphosphat, in einer Menge von 0,1 bis 1,0 Gew.%; Anfärbestoffe, wie z.B. Fluorescein Natriumsalz, in einer Menge von etwa 0,1 bis 1,0 Gew.%; Pflegestoffe, wie zum Beispiel Pflanzen-und Kräuterextrakte, Protein- und Seidenhydrolysate, Lanolinderivate, in einer Menge von 0,1 bis 5 Gew.%; physiologisch verträgliche Silikonderivate, wie z.B. flüchtige oder nicht-flüchtige Silikonöle oder hochmolekulare Siloxanpolymere in einer Menge von 0,05 bis 20 Gew.%; Lichtschutzmittel, Antioxidantien, Radikalfänger, Antischuppenwirkstoffe in einer Menge von etwa 0,01 bis 2 Gew.%; Glanzgeber, Vitamine, Weichmacher, Kämmbarkeitsverbesserer und rückfettende Agenzien.

### Herstellverfahren

Ein Haarbehandlungsmittels in Form eines zweiphasigen Gels, wobei die eine Phase aus Partikeln eines festen und formstabilen Gels besteht und die andere Phase eine fluide Matrix für die festen Partikel ist und wobei die fluide Phase mindestens einen mehrwertigen Alkohol oder mindestens einen flüssigen Polyether eines mehrwertigen Alkohols enthält, kann erfindungsgemäß hergestellt werden, indem zuerst ein festes, formstabiles Gel hergestellt wird, dieses feste Gel mechanisch in einzelne Partikel zerkleinert wird und anschließend zu diesen Partikeln eine mindestens einen mehrwertigen Alkohol oder mindestens einen flüssigen Polyether eines mehrwertigen Alkohols enthaltende Flüssigkeit zugefügt wird.

Die Herstellung der festen Gelphase kann erfolgen, indem zunächst in einem wässrigen Lösungsmittel Carrageenan sowie gegebenenfalls weitere haarkosmetische Wirk- und Hilfsstoffe gelöst werden, wobei die Menge des Gelbildners sowie Art und Mengen der weiteren haarkosmetischen Wirk- und Hilfsstoffe so gewählt sind, dass sich ein formstabiles, festes Gel ausbilden kann. Falls der Gelbildner oder die Zusatzstoffe bei Raumtemperatur nicht vollständig löslich sind, wird zum Lösen der Stoffe erwärmt, z.B. auf ca. 40-80°C. Anschließend wird die Lösung solange stehen gelassen, bis das Gel fest wird. Dieser Prozess wird vorteilhafterweise durch zusätzliche externe Abkühlung auf mindestens 50-55°C oder darunter beschleunigt. Anschließend wird das feste Gel mittels eines geeigneten Schneid- oder Stanzwerkzeugs in Partikel zerkleinert. Die Partikelgröße (mittlerer Durchmesser) beträgt vorzugsweise 0,1 bis 10 mm, besonders bevorzugt 0,5 bis 5, insbesondere von 1 bis 5 mm. Diesen Partikeln wird die flüssige Matrix zugesetzt und gerührt bis sich eine homogene Verteilung der festen Gelpartikel in der flüssigen Matrix ergibt.

### Augführungsform mit Gehalt an Treibmitteln

In einer besonderen Ausführungsform enthält das erfindungsgemäße Mittel zusätzlich mindestens ein Treibmittel. Gegenstand der Erfindung ist daher auch ein Haarbehandlungsprodukt, bestehend aus (a) einer druckfesten Verpackung, welche (b) ein treibmittelhaltiges Haarbehandlungsmittel enthält, sowie (c) einer Vorrichtung zum Versprühen oder Verschäumen des Haarbehandlungsmittels, wobei das Haarbehandlungsmittel in Form eines zweiphasigen Gels vorliegt, wobei die eine Phase aus Partikeln eines festen und formstabilen Gels besteht und die andere Phase eine fluide Matrix für die festen Partikel ist und wobei die fluide Phase mindestens einen mehrwertigen Alkohol oder mindestens einen flüssigen Polyether eines mehrwertigen Alkohols enthält und wobei das Mittel zusätzlich mindestens ein Treibmittel enthält. Das Mittel ist also dreiphasig. Es enthält eine erste (feste) Phase, gebildet aus Partikeln eines festen und formstabilen Gels. Eine zweite (flüssige) Phase dient als fluide Matrix für die festen Gelpartikel und das Treibmittel bildet eine dritte (gasförmige) Phase. Das Gewichtsverhältnis der Wirkstoffzusammensetzung gebildet aus Gelpartikeln und fluider Matrix zur Treibmittelmenge ist vorzugsweise 60 bis 80 Gew.%, besonders bevorzugt 65 bis 70 Gew.% Wirkstoffzusammensetzung zu 20 bis 40 Gew.%, besonders bevorzugt 30 bis 35 Gew.% Treibmittel.

Geeignete Treibmittel sind beispielsweise niedere Alkane, wie z.B. Propan, n-Butan, Isobutan oder auch deren Gemische sowie Dimethylether oder Fluorkohlenwasserstoffe wie F 152a (1,1-Difluorethan) oder F 134 (Tetrafluorethan) sowie ferner bei den in Betracht kommenden Drücken gasförmig vorliegende Treibmittel, wie beispielsweise N₂, N₂O und CO₂ sowie Gemische der vorstehend genannten Treibmittel. Besonders bevorzugt ist Dimethylether. Besonders vorteilhaft ist es, wenn zu dem primären Treibmittel noch eine geringe Menge, z.B. 1 bis 10 Gew.% Pentan zugesetzt wird.

Das treibmittelhaltige Haarbehandlungsprodukt weist als zusätzliche Komponente eine Vorrichtung zum Versprühen oder Verschäumen der Zusammensetzung auf. Hierfür kann ein handelsüblicher Sprüh- oder Schaumkopf verwendet werden. Die treibmittelhaltige Zusammensetzung wird in einer druckfesten Verpackung abgefüllt. Als Verpackungsmaterial können die hierfür üblichen metallischen Materialien wie Aluminium oder Weissblech verwendet werden aber auch Glas oder druckfeste Kunststoffe wie Polyethylen, Polypropylen oder Polyethylenterephtalat. Besonders bevorzugt werden klare, durchsichtige Materialien, insbesondere Glas und durchsichtige Kunststoffe. Die Herstellung erfolgt, indem zunächst die zweiphasige Zusammensetzung aus festen Gelpartikeln und fluider Matrix wie oben beschrieben hergestellt wird und anschließend diese Masse zusammen mit dem Treibmittel in der druckfesten Verpackung abgefüllt wird.

Die treibmittelhaltige Ausführungsform weist die besonderen Vorteile auf, dass die Verreibbarkeit der Zusammensetzung in der Hand und die Verteilbarkeit auf dem Haar erheblich verbessert sind. Außerdem wird ein besonderer sensorischer, kühlender Effekt erzielt.

Die nachfolgenden Beispiele sollen den Gegenstand der Erfindung näher erläutern. Die in den Beispielen angegebenen Polymergehalte beziehen sich jeweils auf den Feststoffgehalt.

### Beispiele

### Beispiel 1

| | |
|---|---|
| 1,6 g | Sea Kem^{®} CM 611 (Carrageenan und Dextrose, FMC Corp.) |
| 2,5 g | Luviset^{®} CA 66 (Vinylacetat/Crotonsäure Copolymer, BASF) |
| 0,27 g | Aminomethylpropanol (95%ig) |
| 2,5 g | Pure Thix^{®} TX 1442 (Polyether-1, Süd-Chemie, United Catalysts) |
| 20 g | Ethanol |
| ad 100 g | Wasser |

Die genannten Bestandteile werden zur Herstellung der festen Gelphase unter Erwärmen auf ca. 70 °C in dem Lösungsmittel gelöst. Anschließend wird durch Stehenlassen bis auf Raumtemperatur abgekühlt, wobei sich das Gel verfestigt. Das feste Gel wird mit Hilfe eines Spatels in kleine unregelmäßige Stücke mit einem mittleren Durchmesser von 2 bis 4 mm zerkleinert. Zu 72 Gew.% der zerkleinerten festen Gelphase werden 28 Gew.% Glycerin zugegeben und vorsichtig umgerührt.

### Beispiel 2

Es werden zerkleinerte feste Gelpartikel gemäß Beispiel 1 hergestellt. Zu 67 Gew.% der festen Gelpartikel werden 33 Gew.% PEG-4 (Polyglykol 200) zugegeben und vorsichtig umgerührt.

### Beispiel 3

| | |
|---|---|
| 1,5 g | Sea Kem^{®} CM 611 (Carrageenan und Dextrose, FMC Corp.) |
| 30 g | Ethanol |
| ad 100 g | Wasser |

Die genannten Bestandteile werden zur Herstellung der festen Gelphase unter Erwärmen auf ca. 70 °C in dem Lösungsmittel gelöst. Anschließend wird durch Stehenlassen bis auf Raumtemperatur abgekühlt, wobei sich das Gel verfestigt. Das feste Gel wird mit Hilfe eines Spatels in kleine unregelmäßige Stücke mit einem mittleren Durchmesser von 2 bis 4 mm zerkleinert. Zu 67 Gew.% der zerkleinerten festen Gelphase werden 33 Gew.% Glycerin zugegeben und vorsichtig umgerührt.

### Beispiel 4

Es werden zerkleinerte feste Gelpartikel gemäß Beispiel 3 hergestellt. Zu 67 Gew.% der festen Gelpartikel werden 33 Gew.% PEG-4 (Polyglykol 200) zugegeben und vorsichtig umgerührt.

### Beispiel 5

65 Gew.% der Zusammensetzung von Beispiel 1 werden mit
35 Gew.% Dimethylether in einer Aerosoldose abgefüllt.

### Beispiel 6

70 Gew.% der Zusammensetzung von Beispiel 2 werden mit
30 Gew.% Dimethylether in einer Aerosoldose abgefüllt.

### Beispiel 7

70 Gew.% der Zusammensetzung von Beispiel 3 werden mit
30 Gew.% Dimethylether in einer Aerosoldose abgefüllt.

Bei sämtlichen Beispielen blieb die zweiphasige Konsistenz aus festen Gelpartikeln und flüssiger Matrix auch nach einer Woche Lagerung bei 40°C erhalten.

Die Präparate sind gut auf dem Haar verteilbar. Sie verleihen der Frisur Modellierbarkeit, flexiblen Halt und Stabilität und geben dem Haar einen Eindruck von Feuchtigkeit und Glanz.

## Patentansprüche

1. Haarbehandlungsmittel in Form eines zweiphasigen Gels, wobei die eine Phase aus Partikeln eines festen und formstabilen Gels besteht und Carrageenan als Gelbildner enthält und die andere Phase eine fluide Matrix für die festen Partikel ist und wobei die fluide Phase mindestens einen mehrwertigen Alkohol oder mindestens einen flüssigen Polyether eines mehrwertigen Alkohols enthält, **dadurch gekennzeichnet, dass** der mittlere Durchmesser der festen Gelpartikel mindestens 1 mm beträgt und/oder dass das Haarbehandlungsmittel zusätzlich mindestens einen Stoff enthält, der ausgewählt ist aus haarfestigenden nichtionischen, anionischen, zwitterionischen und amphoteren Polymeren, amphiphilen Assoziativverdickern, Cellulosederivaten, Zuckern und Treibmitteln, wobei die Zucker Mono- oder Disaccharide sind.

2. Mittel nach Anspruch 1, **dadurch gekennzeichnet, dass** der Widerstand der festen Gelpartikel gegen Kompression unter Normbedingungen (20°C, 65% rel. Luftfeuchte) mindestens 0,15 N beträgt, gemessen durch Penetration des festen Gels mit einem zylindrischen Stempel eines Durchmessers von 8 mm, wobei die Penetration mit einer Geschwindigkeit von 0,5 mm/sec bis zu einer Kompressionstiefe von 1 mm erfolgt und anschließend der Stempel mit einer Geschwindigkeit von 0,5 mm/s wieder zurückgeführt wird.

3. Mittel nach Anspruch 1 oder 2, **dadurch gekennzeichnet, dass** der mehrwertige Alkohol oder der flüssige Polyether der fluiden Matrix ausgewählt ist aus Akylenglykolen, Glycerin und flüssigen Polyalkylenglykolen.

4. Mittel nach einem der vorhergehenden Ansprüche, **dadurch gekennzeichnet, dass** die festen Gelpartikel einen Gehalt aufweisen an einer Kombination von
(A) Carrageenan und
(B) mindestens einem haarfestigenden Polymer
in einer wasserhaltigen Grundlage, wobei die Komponenten (A) und (B) in solchen Mengen enthalten sind, dass die Partikel bei Raumtemperatur in Form eines festen, formstabilen Gels vorliegen.

5. Mittel nach einem der vorhergehenden Ansprüche, **dadurch gekennzeichnet, dass** es sich bei dem Carrageenan um kappa-Carrageenan oder um ein kappa-Carrageenan enthaltendes Carrageenan-Gemisch handelt.

6. Mittel nach einem der vorhergehenden Ansprüche, **dadurch gekennzeichnet, dass** das haarfestigende Polymer ausgewählt ist aus anionischen, nichtionischen und amphoteren Polymeren.

7. Mittel nach Anspruch 6, **dadurch gekennzeichnet, dass** das haarfestigende Polymer ausgewählt ist aus vernetzten oder unvernetzten Vinylacetat/Crotonsäure Copolymeren, partialveresterten Copolymeren zwischen Vinylmethylether und Maleinsäureanhydrid, Terpolymeren aus Acrylsäure, Alkylacrylat und N-Alkylacrylamid, Terpolymeren aus Vinylacetat, Crotonat und Vinylalkanoat, Copolymeren aus Acryl- oder Methacrylsäure und Acryl- oder Methacrylsäurealkylestern, Polyvinylpyrrolidonen und Vinylpyrrolidon/Vinylacetat Copolymeren sowie Polymeren aus Alkylacrylamid, Alkylaminoalkylmethacrylat und zwei oder mehr Monomeren bestehend aus Acrylsäure, Methacrylsäure oder deren Estern, wobei mindestens eines dieser Monomere eine Säuregruppe enthält.

8. Mittel nach einem der vorhergehenden Ansprüche, **dadurch gekennzeichnet, dass** das Carrageenan in einer Menge von mindestens 1 Gew.% bis kleiner 2 Gew.% und das haarfestigende Polymer in einer Menge von 0,5 bis 15 Gew.% vorliegt.

9. Mittel nach einem der vorhergehenden Ansprüche, **dadurch gekennzeichnet, dass** die feste Gelphase zusätzlich mindestens einen die Festigkeit der festen Gelpartikel erhöhenden Stoff enthält, ausgewählt aus Calciumonen und Kaliumionen, vorzugsweise in einer Menge von 0,2 bis 1 Gew.%, und/oder mindestens 15 Gew.% mindestens eines ein- oder mehrwertigen C1- bis C5-Alkohols.

10. Mittel nach einem der vorhergehenden Ansprüche, **dadurch gekennzeichnet, dass** die festen Gelpartikel einen Gehalt aufweisen an einer Kombination von
(A) Carrageenan und
(B) mindestens 15 Gew.% mindestens eines ein- oder mehrwertigen C1- bis C5-Alkohols
in einer wasserhaltigen Grundlage.

11. Mittel nach einem der vorhergehenden Ansprüche, **dadurch gekennzeichnet, dass** es zusätzlich Xanthan Gum enthält.

12. Mittel nach einem der vorhergehenden Ansprüche, **dadurch gekennzeichnet, dass** der Zucker ausgewählt ist aus Glukose, Galaktose, Fructose, Maltose, Lactose und Saccharose.

13. Mittel nach einem der vorhergehenden Ansprüche, **dadurch gekennzeichnet, dass** die Menge an festen Gelpartikeln 30 bis 95 Gew.% und die Menge der fluiden Phase 5 bis 70 Gew,% beträgt.

14. Mittel nach einem der vorhergehenden Ansprüche, **dadurch gekennzeichnet, dass** das Treibmittel Dimethylether ist.

15. Haarbehandlungsprodukt, bestehend aus (a) einer druckfesten Verpackung, welche (b) ein treibmittelhaltiges Haarbehandlungsmittel enthält, sowie (c) einer Vorrichtung zum Versprühen oder Verschäumen des Haarbehandlungsmittels, wobei das Haarbehandlungsmittel in Form eines zweiphasigen Gels vorliegt, wobei die eine Phase aus Partikeln eines festen und formstabilen Gels besteht und die andere Phase eine fluide Matrix für die festen Partikel ist und wobei die fluide Phase mindestens einen mehrwertigen Alkohol oder mindestens einen flüssigen Polyether eines mehrwertigen Alkohols enthält und wobei das Mittel zusätzlich mindestens ein Treibmittel enthält.

16. Verfahren zur Herstellung eines Haarbehandlungsmittels in Form eines zweiphasigen Gels, wobei die eine Phase aus Partikeln eines festen und formstabilen Gels besteht und die andere Phase eine fluide Matrix für die festen Partikel ist und wobei die fluide Phase mindestens einen mehrwertigen Alkohol oder mindestens einen flüssigen Polyether eines mehrwertigen Alkohols enthält, **dadurch gekennzeichnet, dass** zuerst ein festes, formstabiles Gel hergestellt wird, dieses feste Gel mechanisch in einzelne Partikel zerkleinert wird und anschließend zu diesen Partikeln eine mindestens einen mehrwertigen Alkohol oder mindestens einen flüssigen Polyether eines mehrwertigen Alkohols enthaltende Flüssigkeit zugefügt wird.

17. Verfahren nach Anspruch 16, **dadurch gekennzeichnet, dass** das Mittel mit einem Treibmittel in einem druckfestem Behälter abgefüllt wird.

18. Verwendung eines Haarbehandlungsmittels in Form eines zweiphasigen Gels, wobei die eine Phase aus Partikeln eines festen und formstabilen Gels besteht und die andere Phase eine fluide Matrix für die festen Partikel ist und wobei die fluide Phase mindestens einen mehrwertigen Alkohol oder mindestens einen flüssigen Polyether eines mehrwertigen Alkohols enthält zur Erzeugung von Halt oder Modellierbarkeit menschlicher Haare.

## Claims

1. Hair treatment composition in the form of a two-phase gel, where the one phase consists of particles of a solid and dimensionally stable gel and comprises carrageenan as gel former, and the other phase is a fluid matrix for the solid particles and where the fluid phase comprises at least one polyhydric alcohol or at least one liquid polyether of a polyhydric alcohol, **characterized in that** the average diameter of the solid gel particles is at least 1 mm and/or that the hair treatment composition additionally comprises at least one substance which is selected from hair-setting nonionic, anionic, zwitterionic and amphoteric polymers, amphiphilic associative thickeners, cellulose derivatives, sugars and propellants, where the sugars are monosaccharides or disaccharides.

2. Composition according to Claim 1, **characterized in that** the resistance of the solid gel particles to compression under standard conditions (20°C, 65% relative humidity) is at least 0.15 N, measured by penetration of the solid gel with a cylindrical punch of diameter 8 mm, where the penetration takes place with a speed of 0.5 mm/sec to a compression depth of 1 mm, and then the punch is returned at a speed of 0.5 mm/s.

3. Composition according to Claim 1 or 2,
**characterized in that** the polyhydric alcohol or the liquid polyether of the fluid matrix is selected from alkylene glycols, glycerol and liquid polyalkylene glycols.

4. Composition according to one of the preceding claims, **characterized in that** the solid gel particles have a content of a combination of
(A) carrageenan and
(B) at least one hair-setting polymer in a hydrous base, where components (A) and (B) are present in amounts such that the particles are present at room temperature in the form of a solid, dimensionally stable gel.

5. Composition according to one of the preceding claims, **characterized in that** the carrageenan is kappa-carrageenan or a carrageenan mixture containing kappa-carrageenan.

6. Composition according to one of the preceding claims, **characterized in that** the hair-setting polymer is selected from anionic, nonionic and amphoteric polymers.

7. Composition according to Claim 6, **characterized in that** the hair-setting polymer is selected from crosslinked or uncrosslinked vinyl acetate/crotonic acid copolymers, partially esterified copolymers between vinyl methyl ether and maleic anhydride, terpolymers of acrylic acid, alkyl acrylate and N-alkylacrylamide, terpolymers of vinyl acetate, crotonate and vinyl alkanoate, copolymers of acrylic acid or methacrylic acid and acrylic or methacrylic acid alkyl esters, polyvinylpyrrolidones and vinylpyrrolidone/vinyl acetate copolymers, and also polymers of alkylacrylamide, alkylaminoalkyl methacrylate and two or more monomers consisting of acrylic acid, methacrylic acid or esters thereof, where at least one of these monomers contains an acid group.

8. Composition according to one of the preceding claims, **characterized in that** the carrageenan is present in an amount of at least 1% by weight to less than 2% by weight and the hair-setting polymer is present in an amount of from 0.5 to 15% by weight.

9. Composition according to one of the preceding claims, **characterized in that** the solid gel phase additionally comprises at least one substance which increases the solidity of the solid gel particles, selected from calcium ions and potassium ions, preferably in an amount of from 0.2 to 1% by weight, and/or at least 15% by weight of at least one monohydric or polyhydric C1- to C5-alcohol.

10. Composition according to one of the preceding claims, **characterized in that** the solid gel particles have a content of a combination of
(A) carrageenan and
(B) at least 15% by weight of at least one monohydric or polyhydric C1- to C5-alcohol
in a hydrous base.

11. Composition according to one of the preceding claims, **characterized in that** it additionally comprises xanthan gum.

12. Composition according to one of the preceding claims, **characterized in that** the sugar is selected from glucose, galactose, fructose, maltose, lactose and sucrose.

13. Composition according to one of the preceding claims, **characterized in that** the amount of solid gel particles is 30 to 95% by weight and the amount of the fluid phase is 5 to 70% by weight.

14. Composition according to one of the preceding claims, **characterized in that** the propellant is dimethyl ether.

15. Hair treatment product consisting of (a) a pressure-resistant packaging which comprises (b) a propellant-containing hair treatment composition, and (c) a device for spraying or foaming the hair treatment composition, where the hair treatment composition is in the form of a two-phase gel, where the one phase consists of particles of a solid and dimensionally stable gel and the other phase is a fluid matrix for the solid particles and where the fluid phase comprises at least one polyhydric alcohol or at least one liquid polyether of a polyhydric alcohol and where the composition additionally comprises at least one propellant.

16. Process for the preparation of a hair treatment composition in the form of a two-phase gel, where the one phase consists of particles of a solid and dimensionally stable gel and the other phase is a fluid matrix for the solid particles and where the fluid phase comprises at least one polyhydric alcohol or at least one liquid polyether of a polyhydric alcohol, **characterized in that** firstly a solid, dimensionally stable gel is prepared, this solid gel is mechanically comminuted into individual particles and then a liquid containing at least one polyhydric alcohol or at least one liquid polyether of a polyhydric alcohol is added to these particles.

17. Process according to Claim 16, **characterized in that** the composition is bottled with a propellant in a pressure-resistant container.

18. Use of a hair treatment composition in the form of a two-phase gel, where the one phase consists of particles of a solid and dimensionally stable gel and the other phase is a fluid matrix for the solid particles and where the fluid phase comprises at least one polyhydric alcohol or at least one liquid polyether of a polyhydric alcohol for generating hold or modelability of human hair.

## Revendications

1. Composition de traitement capillaire sous forme d'un gel en deux phases, une phase étant constituée de particules d'un gel solide et à stabilité dimensionnelle et contenant du carraghénane en tant que générateur de gel et l'autre phase étant une matrice fluide pour les particules solides et la phase fluide contenant au moins un alcool polyhydrique ou au moins un polyéther liquide d'un alcool polyhydrique, **caractérisée en ce que** le diamètre moyen des particules de gel solide est d'au moins 1 mm et/ou en ce que la composition de traitement capillaire en outre contient au moins une substance qui est choisie parmi des polymères non ioniques, anioniques, zwitterioniques et amphotères fixant les cheveux, des épaississants associatifs amphiphiles, des dérivés de cellulose, des sucres et des propulseurs, les sucres étant des mono- ou disaccharides.

2. Composition selon la revendication 1, **caractérisée en ce que** la résistance des particules de gel solides à la compression dans des conditions normalisées (20 °C, 65 % d'humidité relative de l'air) est d'au moins 0,15 N, mesurée par pénétration dans le gel solide d'un poinçon cylindrique d'un diamètre de 8 mm, la pénétration s'effectuant à une vitesse de 0,5 mm/s jusqu'à une profondeur de compression de 1 mm et le poinçon étant ensuite retiré à une vitesse de 0,5 mm/s.

3. Composition selon la revendication 1 ou 2,
**caractérisée en ce que** l'alcool polyhydrique ou le polyéther liquide de la matrice fluide est choisi parmi les alkylèneglycols, le glycérol et les polyalkylèneglycols liquides.

4. Composition selon l'une quelconque des revendications précédentes, **caractérisée en ce que** les particules de gel solides ont une teneur en une association de
(A) carraghénane et
(B)au moins un polymère fixant les cheveux dans une base aqueuse, les composants (A) et (B) étant contenus en quantités telles que les particules à la température ambiante se trouvent sous forme d'un gel solide à stabilité dimensionnelle.

5. Composition selon l'une quelconque des revendications précédentes, **caractérisée en ce que** le carraghénane consiste en carraghénane kappa ou un mélange de carraghénanes contenant du carraghénane kappa.

6. Composition selon l'une quelconque des revendications précédentes, **caractérisée en ce que** le polymère fixant les cheveux est choisi parmi des polymères anioniques, non ioniques et amphotères.

7. Composition selon la revendication 6, **caractérisée en ce que** le polymère fixant les cheveux est choisi parmi des copolymères acétate de vinyle/acide crotonique réticulés ou non réticulés, des copolymères entre l'oxyde de vinyle et de méthyle et l'anhydride maléique, partiellement estérifiés, des terpolymères d'acide acrylique, acrylate d'alkyle et N-alkylacrylamide, des terpolymères d'acétate de vinyle, crotonate et alcanoate de vinyle, des copolymères d'acide acrylique ou méthacrylique et d'acrylates ou méthacrylates d'alkyle, les polyvinylpyrrolidones et des copolymères vinylpyrrolidone/acétate de vinyle ainsi que des polymères d'alkylacrylamide, méthacrylate d'alkylaminoalkyle et de deux ou plus de deux monomères consistant en acide acrylique, acide méthacrylique ou leurs esters, au moins l'un de ces monomères comportant un groupe acide.

8. Composition selon l'une quelconque des revendications précédentes, **caractérisée en ce que** le carraghénane est présent en une quantité d'au moins 1 % en poids à moins de 2 % en poids et le polymère fixant les cheveux est présent en une quantité de 0,5 à 15 % en poids.

9. Composition selon l'une quelconque des revendications précédentes, **caractérisée en ce que** la phase de gel solide en outre contient au moins une substance augmentant la solidité des particules de gel solide, choisie parmi les ions calcium et les ions potassium, de préférence en une quantité de 0,2 à 1 % en poids, et/ou au moins 15 % en poids d'au moins un alcool monohydrique ou polyhydrique en C₁-C₅.

10. Composition selon l'une quelconque des revendications précédentes, **caractérisée en ce que** les particules de gel solide ont une teneur en une association de
(A) carraghénane et
(B) au moins 15% en poids d'au moins un alcool mono- ou polyhydrique en C₁-C₅
dans une base aqueuse.

11. Composition selon l'une quelconque des revendications précédentes, **caractérisée en ce qu'**elle contient en outre de la gomme xanthane.

12. Composition selon l'une quelconque des revendications précédentes, **caractérisée en ce que** le sucre est choisi parmi le glucose, le galactose, le fructose, le maltose, le lactose et le saccharose.

13. Composition selon l'une quelconque des revendications précédentes, **caractérisée en ce que** la quantité de particules de gel solide va de 30 à 95 % en poids et la quantité de la phase fluide va de 5 à 70 % en poids.

14. Composition selon l'une quelconque des revendications précédentes, **caractérisée en ce que** le propulseur est l'éther diméthylique.

15. Produit de traitement capillaire, consistant en
(a) un emballage tenant la pression, qui contient
(b) une composition de traitement capillaire contenant un propulseur, ainsi que (c) un dispositif pour la pulvérisation ou la transformation en mousse de la composition de traitement capillaire, la composition de traitement capillaire se trouvant sous forme d'un gel en deux phases, une phase étant constituée de particules d'un gel solide et à stabilité dimensionnelle et l'autre phase étant une matrice fluide pour les particules solides et la phase fluide contenant au moins un alcool polyhydrique ou au moins un polyéther liquide d'un alcool polyhydrique et la composition en outre contenant au moins un propulseur.

16. Procédé pour la préparation d'une composition de traitement capillaire sous forme d'un gel en deux phases, une phase étant constituée de particules d'un gel solide et à stabilité dimensionnelle et l'autre phase étant une matrice fluide pour les particules solides et la phase fluide contenant au moins un alcool polyhydrique ou au moins un polyéther liquide d'un alcool polyhydrique, **caractérisé en ce qu'**on prépare d'abord un gel solide à stabilité dimensionnelle on fragmente mécaniquement ce gel solide en particules individuelles et ensuite on ajoute à ces particules un liquide contenant au moins un alcool polyhydrique ou au moins un polyéther liquide d'un alcool polyhydrique.

17. Procédé selon la revendication 16, **caractérisé en ce que** la composition est introduite avec un propulseur dans un récipient tenant la pression

18. utilisation d'une composition de traitement capillaire sous forme d'un gel en deux phases, une phase étant constituée de particules d'un gel solide et à stabilité dimensionnelle et l'autre phase étant une matrice fluide pour les particules solides et la phase fluide contenant au moins un alcool polyhydrique ou au moins un polyéther liquide d'un alcool polyhydrique, pour la production de tenue ou d'aptitude au modelage de cheveux humains.
